# EUROPEAN PATENT SPECIFICATION

(11) **EP 2 421 476 B1**
(45) Date of publication and mention of the grant of the patent: **29.04.2020**
(21) Application number: 10727964.8
(22) Date of filing: 19.04.2010
(51) Int. Cl.: A61F 2/36

(54) **HIP PROSTHESIS**
HÜFTPROTHESE
PROTHÈSE DE HANCHE

(30) Priority: 20.04.2009 IT RM20090179
(43) Date of publication of application: 29.02.2012
(73) Proprietor: Biomet Global Supply Chain Center B.V., 3316 LC Dordrecht (NL)
(72) Inventor: Grappiolo, Guido, 17024 Finale Ligute (SV) (IT)
(74) Representative: Mays, Julie
(86) International application number: PCT/IT2010/000168
(87) International publication number: WO 2010/122590

(56) References cited:
- EP-A- 0 141 022
- EP-A- 0 677 281
- EP-A- 0 709 071
- WO-A2-00/59410
- FR-A- 2 868 689

## Description

The present invention relates to a hip prosthesis,

More specifically, the invention concerns a prosthesis permitting avoiding rotation or sinking phenomenons, thus permitting an optimum diaphyseal blocking.

As it is well known, a hip prosthesis is typically comprised of a femur stem, to be inserted within the medullary channel of the femur on which prosthesis must be provided, comprising articulation of the interested hip, a prosthetic neck, at the end of which a head is inserted, which is part of the prosthetic device and is destined to be inserted within a cavity usually comprised of polyethylene. Said head is suitable to be placed within the cotyloid cavity of the hip iliac bone, at the level of which it is usually inserted a metallic cotyle having a suitable shape.

An example of a hip prosthesis according to known technique is described in the US Patent Application n° US 2006/0190092.

A further example of known prosthesis is described in European Patent EP 0 985 385 B1, wherein femur stem has longitudinal reliefs provided with a particular spacing in order to permit a better fixing. However, said reliefs have a cross-section not permitting an optimum osteo-integ ration.

An example of hip prosthesis known from patent application EP141022A, which forms part of the state of the art.

One of the main problems of hip prostheses according known art is detachment of femur stem, or removal of bone capital.

These limits make it difficult possible revisions and maintenance of the same prosthesis; furthermore, standard length of femur stems makes it complicated their use with mini-invasive techniques aiming respecting muscle tissues, tendons and teguments.

Moreover, standard prosthesis, if shortened, do not fully eliminate serious phenomenons of prosthesis rotation within the femur channel and stem sinking, that must be prevented since the implantation of the same prosthesis.

A further limitation of the known prosthesis is that they do not permit its introduction within medullary channel, since greater trochanter base must be removed, thus reducing preservation of bone tissue. Moreover, as it is well known, femur is subjected to a bending moment on its proximal portion. Therefore, during the post-implantation step, in case stems are introduced with a recto or acute angle, it is necessary, for surgical dimensions reasons, inserting femur stem after having carried out osteotomy of femur neck and shoulder. This creates a discontinuity of the bone/prosthesis structure, since a space is created close to the contact point with prosthesis.

It is also known that femur channel does not have a perfectly circular cross-section, but rather a quite oval cross-section, with a narrower or wider curvature is provided in the medial position, while larger curvature is provided in lateral position. Broken trapezoidal cross-section usually used for known femur stem, has the advantage of increasing stereo-stability, but has three main drawbacks: (i) its shape imposes four point contacts (always on the cross-section) with femur cortical bone; (ii) stem volume is often under-dimensioned with respect to femur channel; (iii) length of this kind of stem is usually too long, in order to increase its stability.

Circular section stems too are not anatomically suitable to the femur channel shape.

In view of the above, it is therefore object of the present invention that of providing a hip prosthesis that can solve the above technical drawbacks, permitting an optimum osteo-integration.

It is also object of the present invention that of suggesting a hip prosthesis permitting obtaining a solid blocking within femur since the realisation of the implantation.

Another object of the invention is that of suggesting a hip prosthesis provided with a rather short femur stem, but that can distribute mechanical tension peaks on bone tissue of femur with prosthesis ensuring initial stability notwithstanding reduction of lateral dimension, so as to preserve bone capital also at the greater trochanter level.

The present invention is defined in claim 1. Further embodiments are defined in dependent claims. It is therefore specific object of the present invention a hip prosthesis comprising a femoral stem that can be inserted within the medullar channel of a patient femur, the medial side of said femoral stem having, in the proximal portion, the shape of an arc, said femoral stem being provided with substantially longitudinal lateral reliefs, so that said stem can be fixed to the bone tissue by pressure, and a prosthetic neck, provided with means for coupling with a morse cone type spherical head, said prosthesis being characterised in that the cross section of said longitudinal reliefs has an apex portion), having the lateral walls included within a first angle, and a base portion having the lateral walls included within a second angle, which is minor or equal to said first angle, and in that grooves are provided between said longitudinal reliefs, that can be homogeneously filled with spongious bone tissue.

Always according to the invention, said femoral stem has a front side and a rear side on which said reliefs are arranged.

Still according to the invention, said femoral stem has a main axis, said main axis being the rectilinear line connecting the apex of the distal end of said femoral stem and the point in which said femoral stem joins with said prosthetic neck, and in that the main relief of each front and rear side, arranged in correspondence with said main axis, has an height greater than the other adjacent longitudinal reliefs.

Furthermore according to the invention, the edges of said distal end are so shaped to obtain bevellings.

Always according to the invention, cross section of said longitudinal reliefs has an inclination of its top with respect to the symmetry axis of said prosthesis different to the inclination with respect to the body of the femoral stem, so as to improve the penetration of said femoral stem and its eventual removability.

Still according to the invention, said grooves have a cross section having a rounded concavity, in such way that they can be homogeneously filled with spongious bone tissue.

Advantageously according to the invention distance between the top of said longitudinal reliefs is constant, preferably equal to 4.5 mm.

Furthermore according to the invention, said femoral stem has a shape tapered towards the distal end.

Preferably according to the invention, said stem has, on its the lateral side, an upper bevelling or shoulder, which has a smooth surface, shaped in such way to preserve the greater trochanter.

Always according to the invention, said shoulder comprises lateral joint surfaces, with said front faces and said rear face, said lateral joint surfaces being preferably rounded.

Still according to the invention, said prosthesis comprises upper longitudinal lateral reliefs and lower longitudinal lateral reliefs, and relevant grooves, arranged on the lateral side, so that: said upper longitudinal lateral reliefs starting substantially from an intermediate point of said shoulder and reaching a portion of the femur neck; and said lower longitudinal lateral reliefs starting substantially from said intermediate point of said shoulder as far a portion of said femoral stem.

Furthermore according to the invention, said prosthetic neck has a longitudinal axis intersecting said main axis of said femoral stem by an angle comprised between 133° and 122°, preferably equal to 133° and 122°.

Advantageously according to the invention, arc of said proximal end has a curvature that substantially comprises the average curvature of the femur of a human being.

Always according to the invention, said femoral stem has a polygonal cross section.

Still according to the invention, said femoral stem comprises at the above a hollow, having an insertion bevelling for inserting a tool, and an internal threaded portion for coupling said tool.

Furthermore according to the invention, said stem can have a substantially trapezoidal cross-section, preferably an isosceles trapezoidal cross-section, the minor base of which is faced toward said medial face and the longer base of which is faced toward said lateral face, angles of said trapezium being bevelled angles.

Present invention will be now described for illustrative, but not limitative purposes, according to its preferred embodiments, with particular reference to the figures of the enclosed drawings, wherein:
figure 1 shows a front view of femur stem of hip prosthesis according to the present invention;
figure 2 shows a medial view of femur stem of hip prosthesis according to figure 1;
figure 3 shows a section view taken along line J-J' of hip prosthesis according to figure 1;
figure 4 shows different steps of introduction of hip prosthesis within endo medullary channel of a femur;
figure 5 shows a plan view of prosthesis of figure 1;
figure 5a shows a particular of figure 5;
figure 6 shows a section view taken along line A-A' hip prosthesis according to figure 1;
figure 7 shows a particular of hip prosthesis according to figure 5; and
figure 8 is a bottom view of prosthesis according to figure 1.

In the different figures, similar parts will be described by the same references.

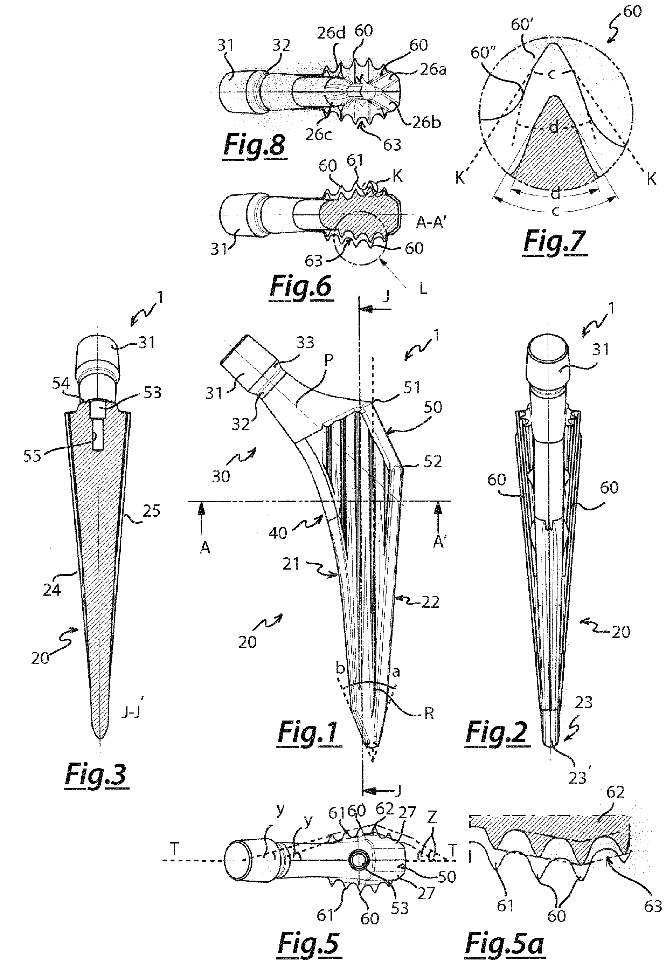

Making reference to figures 1 - 3, it is observed a hip prosthesis 1 according to the invention, comprising a femur stem 20, or endo medullary portion, connected with a prosthetic neck 30, or extra medullary portion.

Prosthetic neck 30 is destined to project from prosthesized femur. Furthermore, said prosthetic neck 30 comprises at its end a morse shaped cone 31, to which a spherical head (not shown in the figures) can be coupled, usually made up of stainless steel, ceramic or cobalt-chrome alloy, suitable to cooperate with a cotyloid cavity. Prosthetic neck 30 also has a cylindrical portion 32. Morse cone 31 joints with said cylindrical portion 32 by a narrowing 33, with an inclination preferably of 45°.

Always making reference to the figures, said femur stem 20 has a medial face 21 and a lateral face 22. Further, said femur stem 20 has a diamond shaped polygonal section.

Length of femur stem 20 is such not to interfere with femur isthmus. In fact, in case femur stem 20, due to its length, fits close to isthmus, stress transfer area would arrive up to that point, and thus too distal from area of insertion of the same stem, causing a transfer from stem-bone interference area from proximal to distal. This strongly condition post-implantation regenerative development, with unavoidable reduction of load on proximal area and overload on distal area close to isthmus.

The above also implies needing of centring the femur stem 20 both on front plane and in lateral stem. Femur stem 20 of prosthesis 1 does not interfere at the distal level and permits, thanks to the shoulder 50, the function of which will be better described in the following, an introduction allowing a bone-prosthesis adhesion in the proximal and trochanter area and not in the distal and diaphyseal area.

Said medial face 21 of said femur stem 20 is joined with prosthetic neck 30 thanks to an upper or proximal portion 40 (provided on the proximal end) of said femur stem 20, with an arc having a curvature substantially including the average curvature of human being.

Instead, lateral face 22 is shaped and has a bevelling realising a kind of shoulder 50 with a smooth surface, obtained between point 52, substantially in correspondence of the half or intermediate point (said intermediate point is individuated by normal line oriented inward the upper or proximal portion 40 joining said point 52) of said arc obtained on the opposed portion of stem 20, i.e. medial face 21, and point 51, wherein said femur stem 20 joints with said prosthetic neck 30.

Said shoulder 50 permits preserving grand trochanter after digging on femur carried out by surgeon for insertion of prosthesis 1, thus also easing insertion of femur stem 20 within said femur medullary channel.

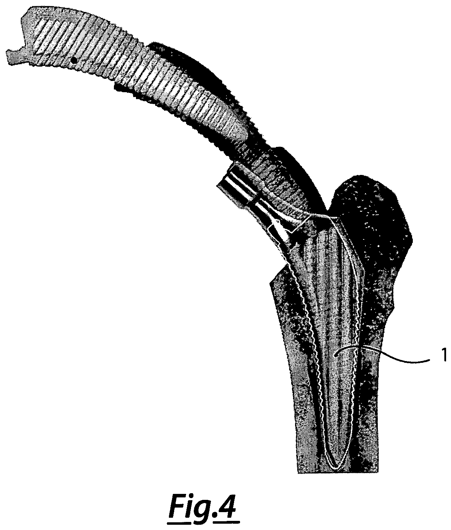

Figure 4 permits observing mode of introduction of prosthesis 1 within endo medullary channel of a femur thanks to the shoulder 50 shape and joint surfaces 27 (see figure 5), in combination with the curved upper or proximal portion 40. Bevelling of lateral proximal portion of prosthesis 1 between continuity plane between neck and femur stem 20 permits introducing it through a neck entrance making a rotation along neck medial cortical. Prosthesis 1 can adhere to the seat obtained by suitable tools, without creating matter spaces and discontinuities that would modify the final stress condition. Said shoulder 50 further promotes a reduced, or even null, aggression of soft parts.

Therefore, morphology of prosthesis 1 in lateral proximal area permits an introduction of the same by rotation with respect to neck 30, without removal of bone tissue from trochanter basis. In fact, taking into consideration that femur is subjected to person weight force, stressing prosthesis 1 femur head, and by traction action of trochanter muscles (medium-gluteus) according to a direction substantially opposed to said person weight force, it is possible imagining application of a bending moment that, in a healthy bone, is transmitted on all the femur proximal portion without discontinuities. Maintaining basis of greater trochanter thus permits maintaining an optimum coupling of prosthesis with bone tissue, although it is stressed by said bending moment. Femur 20 stem is inscribed along front plane within an imaginary triangle, the lateral side of which makes an angle a with a main axis R (that will be better described in the following) substantially placed longitudinally with respect to femur stem 20, while medial side makes an angle *b* with the same main axis R. Said angle *a* and *b* individuates lateral 22 and medial 21 faces of said femur stem 20. Furthermore, based on the above structure, lower or distal end 23 of femur stem 20 has an apex 23'.

The above main axis R is the line passing through the point 51 and apex 23'of distal end 23 of femur stem 20.

Femur stem 20 further has a front face 24 and a rear face 25, on which longitudinal reliefs 60 are provided, following profile of femur stem 20 and substantially extending from proximal end to the distal one of the same femur stem 20.

Making now reference also to figures 5 - 8, it is observed that said longitudinal reliefs 60 do not have all the same height, following a diamond shape profile on its transverse plane.

A main longitudinal relief 61, provided on each one of said front or rear surface 24 or 25, substantially in correspondence of said main axis R of the femur stem 20, is taken into consideration among said reliefs 60. With respect to section line A-A', said main relief 61 is higher than the others.

Height of other longitudinal reliefs 60 decreases both according to the lateral face 22 direction and according to the medial face 21, with a slope different each other.

Distance between peaks of longitudinal reliefs 60 is, in a preferred embodiment, uniform, equal to 4.5 mm.

Longitudinal reliefs 60 are preferably placed on a base 62, having the same profile (slope) of peak of said longitudinal reliefs.

Particularly, making reference to figure 5, it is possible observing angles defining transverse geometry of longitudinal reliefs 60, which are:
- prominence angle z, corresponding to intersection of front-rear angle T plane with lines (dashed lines in the figure) joining peaks of main longitudinal relief 61 with peak of longitudinal reliefs 60 adjacent according to lateral direction, lines laying on or being parallel to the transverse plane A-A';
- full section angle y, corresponding to intersection of said plane T with lines joining peak of main longitudinal relief 61 with peak of longitudinal reliefs 60, adjacent along medial direction, said lines laying on or being parallel to the transverse plane A-A'.

Said longitudinal reliefs 60 permit, increasing torque resistance, reducing femur stem 20 length with respect to known prosthesis, permitting a mini-invasive insertion of the same metaphyseal "press-type" kind, i.e. based on anchoring femur stem 20 with femur bone tissue by friction.

Furthermore, said anchoring is also eased by profile of hip prosthesis 1, since it permits reducing length of distal portion of the femur stem 20.

Grooves 63 are provided between longitudinal reliefs 60, each one having its own ray.

Instead, profile of lugs 60 along transverse plane has a double slope, defined by a first and a second wall opening angle *c* and *d*, different each other, the shaping of which is suitable to differently distribute contact forces with bone tissue. In other words, transverse section of each one of said longitudinal reliefs 60 or 61 has an apex portion 60' with lateral walls inscribed within said first angle *c*, and a base portion 60" having lateral walls inscribed within a second angle *d*, minor or equal to said first angle *c*.

Said walls therefore have two different convergence angles (one angle c and the other one angle *d*), exerting two functions:
- peak of lugs 60 permits an optimum penetration within spongy bone tissue;

Base of each lug 60, varying its slope and joining at the bottom (throat 63), permits filling spaces between the same lugs 60 with spongy tissue, in a homogeneous and full way. In fact, said bone tissue smoothly slides along lug 60 or 61 wall, without smooth or sharp discontinuities.

In other words, said cross section of longitudinal reliefs 60 is suitable to permit to bone tissue to homogeneously and fully (i.e. with a substantially uniform density) fill throats 30.

Difference of said angles c and d per mits increasing bone-prosthesis contact surface, as well as reducing prosthesis 1 volume. Moreover, contact surface increases and transfer of load to bone tissue improves. In fact, stress condition of bone tissue, deriving from prosthesis implantation 1 is strongly variable conditioning outcome of articular reconstruction.

Moreover, one of variables conditioning regeneration of bone tissue is its stress condition. All stresses (stresses and deformations) applied on the same must be within such ranges to prevent under-stresses, so that bone re-absorption would occur; or over-stresses, so that hypertrophism would occur, i.e. not controlled tissue growing, caused by request of tissue to balance overstressing; or even reabsorption caused by incapability of supporting stress condition (biologic collapse).

Load distribution is therefore very important for success of prosthetic implant. Increase of contact surface creates a reduction of load transferred for surface unity and thus a reduction of the over-stresses risk, which is highly probable in the lug 60 area.

Finally, difference between angles *c* and *d* also permits obtaining, with the same penetration of lugs 60 and 61 (lug height), a reduction of prosthesis 1 volume. The above permits preserving the highest bone tissue possible.

Lugs 10 and 11 are joined with prosthesis base 62 according to a radiused mode, thus permitting, as already said, homogeneous collection of bone tissue within throats 63 or interspaces, stimulating regeneration by a uniform stressing of the same tissue. In other words, bone tissue, thanks to said joint permits to the bone tissue housing up to the base (bottom) of throats 63 of lugs 60, thus avoiding discontinuities that would not permit homogeneously collect bone tissue.

It must be taken into consideration that joint ray permits having a direction of pressure exerted from prosthesis on tissue and vice versa (action and reaction principle) varying its direction. This influences on stimulation of regeneration, since increase possibility that total stress condition, generated by stresses intensity and direction, is such to be included within a suitable range. Furthermore, it is to be taken into consideration that, with reference to the lateral plane (the one indicated in figures 1 - 3 by section line J-J), inclination of reliefs 60, 61 peak, with respect to symmetry vertical axis of said prosthesis 1 is different with respect to the one of the femur stem 20 body, in order to improve penetration of femur stem 20 and possibly its removal.

Thanks to the above anchoring and structure, hip prosthesis 1 according to the invention remarkably limits sinking phenomenon and increases torque resistance of prosthesis 1, since longitudinal reliefs 60, and particularly main longitudinal relief 61, distribute along the whole prosthetic body.

Furthermore, configuration of longitudinal reliefs 60 as defined in the above, permits a double "press-fit" coupling, which, in the cortical-spongious and subjected to pressure area permits a better integration with bone tissue, thanks to the action of uniform stresses promoting long term bone remodelling. In fact, femur stem 20 according to the present invention can be implanted without cement. In fact, its surface promotes anchoring to bone tissue thanks to a preferred rugosity associated with a specific treatment of titanium, material preferably comprising prosthesis.

In a preferred embodiment (not shown in the figures), hip prosthesis 1 comprises upper lateral longitudinal reliefs and lower lateral longitudinal reliefs, and corresponding throats, provided on the lateral face 22, in such a way that:
- said upper lateral longitudinal reliefs substantially start from an intermediate point of the shoulder 50 up to reaching a portion of the femur neck 30; and
- said lower lateral longitudinal reliefs substantially start from an intermediate point of the shoulder 50 up to reaching a portion of the femur stem 20.

Cross section of femur stem 20, in a preferred embodiment, preferably has a trapezoidal shape.

Corners of the distal end are preferably shaped, so as to obtain suitable bevellings, indicated by reference numbers 26a, 26b, 26c and 26d.

Said bevellings 26a, 26b, 26c and 26d improve congruence of femur stem 20 with respect to endo medullary channel (without any other sacrifice of bone tissue) and, in case it is necessary removal, ease saving bone tissue.

Furthermore, as anticipated, joint surfaces 27 (see figure 5) are provided in correspondence of the proximal part of shoulder 50, permitting a better adhesion of prosthesis 1 to cortical channel morphology.

Femur stem 20 thus has a cross section with a trapezoidal base, but corners of said trapezium (joint surfaces 27) are bevelled in their lateral part, and rounded in their medial part. Said features permit to the femur stem 20 fully filling femur channel, to have a wide resting on lateral cortical bone and mainly on medial cortical bone, and finally to have a rotation stability.

Stereo-stability is moreover increased by different heights of reliefs 60 (with respect to cross section) on front and rear faces.

In consideration of femur substantially oval natural cross section (wider curvature of lateral portion), main longitudinal relief 61 is preferably positioned in lateral part of said faces. Main longitudinal relief 61 also offers a further resting point on cortical bone. In this case, it is not a surface contact, but a point contact.

Secondary longitudinal reliefs 60 and front and rear faces of femur stem 20 body are zones in contact with spongy tissue. Distribution of contact zones between spongy tissue and cortical bone improves distribution of loads and at the same time stimulates spongy and cortical tissue, in order to promote osteo-integration.

Proximal portion 40 (arc) joining prosthetic neck 30 with femur stem 20 does not have bevellings, but rather joints (rounded).

In order to make it flexible the use of the hip prosthesis 1, a first embodiment of prosthetic neck 30 has axis P intersecting said main axis R of femur stem 20, with a cervical - diaphyseal angle of 133° (standard gamma), while in a second embodiment, said angle is equal to 122° (varus gamma). This permits adhering to the different human morphotypes. Said angles enclose standard deviation of neck varus-valgus physiological angle, thus optimising search of articular rotation centre.

In other words, it is possible using two types of prosthetic necks 30, which have different measures of said cervical - diaphyseal angle.

Reconstruction of prosthesized hip anatomy is very important for success of arthroplasia of the same hip. Principle on which construction of gamma of this prosthesis 1 or implant is based permits covering a wide range of leg offsets and lengths.

Hip prosthesis 1 according to the present invention permits using different sizes of the femur stem 20, in order to obtain offset and length of femur neck more suitable to patient morphology, not only choosing the head, but also choosing size of prosthesis 1.

Size distribution is realised in such a way that femur stem 20 remains similar from size to size, while prosthetic neck 30, i.e. extra medullary portion, varies in terms of length and offset.

Particularly, angles a and b, determining medial 21 and lateral 22 faces, remain uniform while sizes vary. This implies that femur stem 220 is similar as far as their shape and dimensions are concerned, while sizes vary. Thus, it is possible choosing offset and length of prosthetic neck 30, choosing a size rather than another one, within a limited range, maintaining the same diaphyseal surgical preparation.

Above prosthesis 1, a groove 53 is obtained, permitting placing femur stem 20 within a femur cavity. Said groove 53 has a bevelling 54 suitable to ease insertion of a tool 56, and also has an threaded inner portion 55, in order to fix said tool 56 for extraction of femur stem 20 in case of revision or withdrawal.

Femur stem 20 of prosthesis according to the present invention can be therefore easily removed without damaging bone, even when it is well Osseo-integrated, thanks to:
- possibility of realising the same with a reduced length, maintaining the same mechanical fixing performances, thanks to the combined action of longitudinal reliefs 60 and 61, and of throats 63;
- its tapered shape thanks to angles a and b, which are distally converging.

An advantage of the present invention is that said hip prosthesis can be indifferently used as cephalic implant, in case of fracture of the femur neck, or it can be integrated within a prosthetic system for total arthroplasia of hip articulation. In the latter case, stem will have to be used along with a femur head and an acetabular prosthesis.

Present invention has been described for illustrative, but not limitative purposes according to its preferred embodiment, but it is to be understood that variations and/or modifications can be introduced by those skilled in the art without departing from the relevant scope, as defined in the enclosed claims.

## Claims

1. Hip prosthesis (1) comprising:
a prosthetic neck (30), provided with means for coupling with a morse cone type spherical head (31),
a femoral stem (20) that can be inserted within the medullar channel of a patient femur, the femoral stem comprising a front face (24), a rear face (25) and lateral (22) and medial (21) faces, the stem including a base (62), the medial face (21) of said femoral stem (20) being joined with the prosthetic neck (30) by a proximal portion (40) having an arcuate shape, said front (24) and rear (25) faces of the femoral stem (20) being provided with substantially longitudinal lateral reliefs (60, 61) on the base (62), said reliefs (60, 61) extending substantially from a proximal end of the femoral stem (20) to a distal end, so that said stem (20) can be fixed to the bone tissue by pressure; said prosthesis (1) **characterized in that**:
the cross section of each of said longitudinal reliefs (60, 61) has a peak portion (60'), having the lateral walls included within a first angle (c), and a base portion having the lateral walls included within a second angle (d), which is less than said first angle (c), and
grooves (63) provided between said longitudinal reliefs (60, 61), that can be homogeneously filled with spongious bone tissue,
said base (62) having the same profile as defined by the peaks of said longitudinal reliefs (60, 61) in a transverse plane (A-A) of the prosthetic femoral stem (20),
said femoral stem (20) having a main axis (R), said main axis (R) being the rectilinear line connecting the apex (23') of the distal end (23) of said femoral stem (20) and the point (51) in which said femoral stem (20) joins with said prosthetic neck (30), and
a main relief (61) of each of the front (24) and rear (25) sides, has a height from a central coronal plane (T) of the femoral stem greater than the other longitudinal reliefs (60).

2. Prosthesis (1) according to claim 1, **characterized in that** the edges of said distal end (23) are so shaped to obtain bevellings (26a, 26b, 26c, 26d).

3. Prosthesis (1) according to anyone of the preceding claims, **characterized in that** cross section of said longitudinal reliefs (60, 61) has an inclination of its top with respect to the symmetry axis of said prosthesis (1) different to the inclination with respect to the body of the femoral stem (20), so as to improve the penetration of said femoral stem (20) and its eventual removability.

4. Prosthesis (1) according to anyone of the preceding claims, **characterized in that** said grooves (63) have a cross section having a rounded concavity, in such way that they can be homogeneously filled with spongious bone tissue.

5. Prosthesis (1) according to anyone of the preceding claims, **characterized in that** the distance between the top of said longitudinal reliefs (60, 61) is constant, preferably equal to 4.5 mm.

6. Prosthesis (1) according to anyone of the preceding claims, **characterized in that** said femoral stem (20) has a shape tapered towards the distal end (23).

7. Prosthesis (1) according to anyone of the preceding claims, **characterized in that** said stem (20) has, on its the lateral side (22), an upper bevelling or shoulder (50), which has a smooth surface, shaped in such way to preserve the greater trochanter.

8. Prosthesis (1) according to claim 7, **characterized in that** said shoulder (50) comprises lateral joint surfaces (27), with said front faces (24) and said rear face (25), said lateral joint surfaces (27) being preferably rounded.

9. Prosthesis (1) according to anyone of claims 7 or 8, **characterized in that** it comprises upper longitudinal lateral reliefs and lower longitudinal lateral reliefs, and relevant grooves, arranged on the lateral side (22), so that:
- said upper longitudinal lateral reliefs starting substantially from an intermediate point of said shoulder (50) and reaching a portion of the femoral neck (30); and
- said lower longitudinal lateral reliefs starting substantially from said intermediate point of said shoulder (50) as far a portion of said femoral stem (20).

10. Prosthesis (1) according to anyone of the preceding claims, **characterized in that** said prosthetic neck (30) has a longitudinal axis (P) intersecting said main axis (R) of said femoral stem (20) by an angle comprised between 133° and 122°, preferably equal to 133° and 122°.

11. Prosthesis (1) according to anyone of the preceding claims, **characterized in that** the arc of said proximal end (40) has a curvature that substantially comprises the average curvature of the femur of a human being.

12. Prosthesis (1) according to anyone of the preceding claims, **characterized in that** said femoral stem (20) has a polygonal cross section.

13. Prosthesis (1) according to anyone of the preceding claims, **characterized in that** said femoral stem (20) comprises at the above a hollow (53), having an insertion bevelling (54) for inserting a tool (56), and an internal threaded portion (55) for coupling said tool.

14. Prosthesis (1) according to anyone of the preceding claims, **characterized in that** said stem has a substantially trapezoidal cross-section, preferably an isosceles trapezoidal cross-section, the minor base of which is faced toward said medial face (21) and the longer base of which is faced toward said lateral face (22), angles of said trapezium being bevelled angles.

## Patentansprüche

1. Hüftprothese (1) umfassend:
einen Prothesenhals (30), der mit Mitteln zum Koppeln mit einem kugelförmigen Kopf vom Morsekegel-Typ (31) versehen ist;
einen Femurschaft (20), der in den Markkanal eines Patientenfemurs eingeführt werden kann, wobei der Femurschaft eine vordere Fläche (24), eine hintere Fläche (25) und seitliche (22) und mittlere (21) Flächen umfasst, wobei der Schaft eine Basis (62) umfasst, wobei die mittlere Fläche (21) des Femurschafts (20) mit dem Prothesenhals (30) durch einen proximalen Abschnitt (40), der eine bogenförmige Form aufweist, verbunden ist, wobei die vordere (24) und die hintere (25) Fläche des Femurschafts (20) mit im Wesentlichen längsverlaufenden seitlichen Reliefs (60, 61) an der Basis (62) versehen sind, wobei sich die Reliefs (60, 61) im Wesentlichen von einem proximalen Ende des Femurschafts (20) zum einem distalen Ende erstrecken, so dass der Schaft (20) durch Druck am Knochengewebe befestigt werden kann; wobei die Prothese (1) **dadurch gekennzeichnet ist, dass**:
der Querschnitt von jedem der längsverlaufenden Reliefs (60, 61) einen Spitzenabschnitt (60'), wobei die Seitenwände in einem ersten Winkel (c) umfasst sind, und einen Basisabschnitt, wobei die Seitenwände in einem zweiten Winkel (d) umfasst sind, der kleiner als der erste Winkel (c) ist, aufweist und
zwischen den längsverlaufenden Reliefs (60, 61) Rillen (63) vorgesehen sind, die homogen mit spongiösem Knochengewebe gefüllt werden können,
wobei die Basis (62) das gleiche Profil aufweist, wie es durch die Spitzen der längsverlaufenden Reliefs (60, 61) in einer Querebene (A-A) des prothetischen Femurschafts (20) definiert ist,
wobei der Femurschaft (20) eine Hauptachse (R) aufweist, wobei die Hauptachse (R) die geradlinige Linie ist, die den Scheitel (23') des distalen Endes (23) des Femurschafts (20) und den Punkt (51) verbindet, in dem sich der Femurschaft (20) mit dem Prothesenhals (30) verbindet, und
ein Hauptrelief (61) von jeder der vorderen (24) und hinteren (25) Seiten eine Höhe von einer zentralen koronalen Ebene (T) des Femurschafts aufweist, die größer als die anderen längsverlaufenden Reliefs (60) ist.

2. Prothese (1) nach Anspruch 1, **dadurch gekennzeichnet, dass** die Kanten des distalen Endes (23) so geformt sind, dass Abschrägungen (26a, 26b, 26c, 26d) erhalten werden.

3. Prothese (1) nach einem der vorhergehenden Ansprüche, **dadurch gekennzeichnet, dass** der Querschnitt der längsverlaufenden Reliefs (60, 61) eine Neigung seiner Oberseite in Bezug auf die Symmetrieachse der Prothese (1) aufweist, die sich von der Neigung in Bezug auf den Körper des Femurschafts (20) unterscheidet, um das Eindringen des Femurschafts (20) und seine eventuelle Entfernbarkeit zu verbessern.

4. Prothese (1) nach einem der vorhergehenden Ansprüche, **dadurch gekennzeichnet, dass** die Rillen (63) einen Querschnitt mit einer abgerundeten Konkavität aufweisen, so dass sie homogen mit spongiösem Knochengewebe gefüllt werden können.

5. Prothese (1) nach einem der vorhergehenden Ansprüche, **dadurch gekennzeichnet, dass** der Abstand zwischen der Oberseite der längsverlaufenden Reliefs (60, 61) konstant ist, vorzugsweise gleich 4,5 mm.

6. Prothese (1) nach einem der vorhergehenden Ansprüche, **dadurch gekennzeichnet, dass** der Femurschaft (20) eine zum distalen Ende (23) hin verjüngte Form aufweist.

7. Prothese (1) nach einem der vorhergehenden Ansprüche, **dadurch gekennzeichnet, dass** der Schaft (20) an seiner seitlichen Seite (22) eine obere Abschrägung oder Schulter (50) aufweist, die eine glatte Oberfläche aufweist, die so geformt ist, dass der Trochanter major erhalten wird.

8. Prothese (1) nach Anspruch 7, **dadurch gekennzeichnet, dass** die Schulter (50) seitliche Gelenkflächen (27) mit den vorderen Flächen (24) und der hinteren Fläche (25) umfasst, wobei die seitlichen Gelenkflächen (27) vorzugsweise abgerundet sind.

9. Prothese (1) nach einem der Ansprüche 7 oder 8, **dadurch gekennzeichnet, dass** sie obere längsverlaufende seitliche Reliefs und untere längsverlaufende seitliche Reliefs und entsprechende Rillen umfasst, die auf der seitlichen Seite (22) angeordnet sind, so dass:
- die oberen längsverlaufenden seitlichen Reliefs im Wesentlichen von einem Zwischenpunkt der Schulter (50) ausgehen und einen Abschnitt des Femurhalses (30) erreichen; und
- die unteren längsverlaufenden seitlichen Reliefs im Wesentlichen von dem Zwischenpunkt der Schulter (50) bis zu einem Abschnitt des Femurschafts (20) ausgehen.

10. Prothese (1) nach einem der vorhergehenden Ansprüche, **dadurch gekennzeichnet, dass** der Prothesenhals (30) eine Längsachse (P) aufweist, die die Hauptachse (R) des Femurschafts (20) unter einen Winkel zwischen 133° und 122°, vorzugsweise gleich 133° und 122° schneidet.

11. Prothese (1) nach einem der vorhergehenden Ansprüche, **dadurch gekennzeichnet, dass** der Bogen des proximalen Endes (40) eine Krümmung aufweist, die im Wesentlichen die durchschnittliche Krümmung des Femurs eines Menschen umfasst.

12. Prothese (1) nach einem der vorhergehenden Ansprüche, **dadurch gekennzeichnet, dass** der Femurschaft (20) einen polygonalen Querschnitt aufweist.

13. Prothese (1) nach einem der vorhergehenden Ansprüche, **dadurch gekennzeichnet, dass** der Femurschaft (20) oben eine Vertiefung (53) umfasst, die eine Einführschräge (54) zum Einführen eines Werkzeugs (56) und ein Innengewindeabschnitt (55) zum Koppeln des Werkzeugs aufweist.

14. Prothese (1) nach einem der vorhergehenden Ansprüche, **dadurch gekennzeichnet, dass** der Schaft einen im Wesentlichen trapezförmigen Querschnitt aufweist, vorzugsweise einen gleichschenkligen trapezförmigen Querschnitt, dessen kleinere Basis zur mittleren Fläche (21) zugewandt ist und dessen längere Basis zur seitlichen Fläche (22) zugewandt ist, wobei die Winkel des Trapezes abgeschrägte Winkel sind.

## Revendications

1. Prothèse de hanche (1) comprenant :
un col prosthétique (30) doté de dispositifs pour l'accouplement avec une tête sphérique du type à cône morse (31),
une tige fémorale (20) pouvant être insérée dans le passage médullaire du fémur d'un patient, la tige fémorale comprenant une face antérieure (24), une face postérieure (25), ainsi que des faces latérale (22) et médiane (21), la tige comprenant une embase (62), la face médiane (21) de ladite tige fémorale (20) étant jointe au col prosthétique (30) par une partie proximale (40) de forme arquée, lesdites faces antérieure (24) et postérieure (25) de la tige fémorale (20) étant dotées d'évidements latéraux substantiellement longitudinaux (60, 61) sur l'embase (62), lesdits évidements (60, 61) s'étendant substantiellement d'une extrémité proximale de la tige fémorale (20) à une extrémité distale, de sorte que ladite tige (20) puisse être fixée, par pression, sur le tissu osseux ;
ladite prothèse (1) étant **caractérisée en ce que** :
la section transversale de chacun desdits évidements longitudinaux (60, 61) présente une partie de crête (60') dont les parois latérales sont inclues au sein d'un premier angle (c), et une partie d'embase dont les parois latérales sont inclues dans un deuxième angle (d) inférieur audit premier angle (c), et
des cannelures (63) pratiquées entre lesdits évidements longitudinaux (60, 61), pouvant être remplis de façon homogène avec un tissu osseux spongieux,
le profil de ladite embase (62) étant identique à celui qui est défini par les crêtes desdits évidements longitudinaux (60, 61) dans un plan transversal (A-A) de la tige fémorale prosthétique (20),
ladite tige fémorale (20) présentant un axe principal (R), ledit axe principal (R) étant la ligne rectiligne raccordant la crête (23') de l'extrémité distale (23) de ladite tige fémorale (20) au point (51) auquel s'effectue le raccordement de ladite tige fémorale (20) audit col prosthétique (30), et
la hauteur d'un évidement principal (61) de chacun des côtés antérieur (24) et postérieur (25) depuis un plan coronal central (T) de la tige fémorale est supérieure à celle des autres évidements longitudinaux (60).

2. Prothèse (1) selon la revendication 1, **caractérisée en ce que** les bords de ladite extrémité distale (23) sont façonnés de façon à réaliser des biseaux (26a, 26b, 26c, 26d).

3. Prothèse (1) selon une quelconque des revendications précédentes, **caractérisée en ce que** l'inclinaison de la partie supérieure de la section transversale desdits évidements longitudinaux (60, 61), relativement à l'axe de symétrie de ladite prothèse (1) est différente de l'inclinaison relative au corps de la tige fémorale (20), ceci afin de renforcer la pénétration de ladite tige fémorale (20) et son amovibilité éventuelle.

4. Prothèse (1) selon une quelconque des revendications précédentes, **caractérisée en ce que** lesdites cannelures (63) présentent une section transversale avec une concavité arrondie, de sorte que celles-ci puissent être remplies de façon homogène avec un tissu osseux spongieux.

5. Prothèse (1) selon une quelconque des revendications précédentes, **caractérisée en ce que** la distance entre la partie supérieure desdits évidements longitudinaux (60, 61) est constante, et égale de préférence à 4,5 mm.

6. Prothèse (1) selon une quelconque des revendications précédentes, **caractérisée en ce que** ladite tige fémorale (20) présente une forme conique vers l'extrémité distale (23).

7. Prothèse (1) selon une quelconque des revendications précédentes, **caractérisée en ce que** ladite tige (20) présente, sur son côté latéral (22), un biseau ou un épaulement supérieur (50), ayant une forme lisse, et façonné de façon à protéger le grand trochanter.

8. Prothèse (1) selon la revendication 7, **caractérisée en ce que** ledit épaulement (50) comprend des surfaces articulaires latérales (27) avec lesdites faces antérieures (24) et lesdites faces postérieures (25), lesdites surfaces articulaires latérales (27) étant de préférence arrondies.

9. Prothèse (1) selon une quelconque des revendications 7 ou 8, **caractérisée en ce qu'**elle comprend des évidements latéraux longitudinaux supérieurs et des évidements latéraux longitudinaux inférieurs, et cannelures correspondantes, agencés sur le côté latéral (22), de sorte que :
- lesdits évidements latéraux longitudinaux supérieurs partent substantiellement d'un point intermédiaire dudit épaulement (50) et atteignent une partie du col du fémur (30) ; et
- lesdits évidements latéraux longitudinaux inférieurs partent substantiellement d'un point intermédiaire dudit épaulement (50) et atteignent une partie de ladite tige fémorale (20).

10. Prothèse (1) selon une quelconque des revendications précédentes, **caractérisée en ce que** le col prosthétique (30) possède un axe longitudinal (P) croisant ledit axe principal (R) de ladite tige fémorale (20) avec un angle compris entre 133° et 122°, de préférence égal à 133° et 122°.

11. Prothèse (1) selon une quelconque des revendications précédentes, **caractérisée en ce que** l'arc de ladite extrémité proximale (40) présente une courbure comprenant substantiellement la courbure moyenne du fémur d'un être humain.

12. Prothèse (1) selon une quelconque des revendications précédentes, **caractérisée en ce que** ladite tige fémorale (20) présente une section transversale polygonale.

13. Prothèse (1) selon une quelconque des revendications précédentes, **caractérisée en ce que** ladite tige fémorale (20) comprend, à sa partie supérieure, un creux (53) possédant un biseau d'insertion (54) pour l'insertion d'un outil (56), et une partie filetée interne (55) pour le couplage dudit outil.

14. Prothèse (1) selon une quelconque des revendications précédentes, **caractérisée en ce que** ladite tige présente une section transversale substantiellement trapézoïdale, de préférence une section transversale trapézoïdale isocèle, dont la base mineure est tournée vers ladite face médiane (21), et dont la base plus longue est tournée vers la face latérale (22), les angles dudit trapèze étant des angles biseautés.
